(19) Europäisches Patentamt
European Patent Office
Office européen des brevets

(11) Publication number: 0 399 148 A2

(12) **EUROPEAN PATENT APPLICATION**

(21) Application number: 90103557.6

(51) Int. Cl.5: C01B 13/32

(22) Date of filing: 23.02.90

The title of the invention has been amended (Guidelines for Examination in the EPO, A-III, 7.3).

(30) Priority: 27.02.89 JP 48168/89
20.03.89 JP 69095/89

(43) Date of publication of application:
28.11.90 Bulletin 90/48

(84) Designated Contracting States:
CH DE FR GB IT LI NL

(71) Applicant: MITSUBISHI RAYON CO., LTD.
3-19, Kyobashi 2-chome Chuo-Ku
Tokyo 104(JP)

(72) Inventor: Mukai, Nobuhiro, c/o Central Research Laboratories
Mitsubishi Rayon Co.,Ltd., 20-1 Miyuki-cho
Otake-shi, Hiroshima-ken(JP)
Inventor: Sasaki, Isao, c/o Central Research Laboratories
Mitsubishi Rayon Co.,Ltd., 20-1 Miyuki-cho
Otake-shi, Hiroshima-ken(JP)
Inventor: Yamamoto, Naoki, c/o Central Research Laboratories
Mitsubishi Rayon Co.,Ltd., 20-1 Miyuki-cho
Otake-shi, Hiroshima-ken(JP)
Inventor: Ige, Hitsohi, c/o Central Research Laboratories
Mitsubishi Rayon Co.,Ltd., 20-1 Miyuki-cho
Otake-shi, Hiroshima-ken(JP)
Inventor: Atarashi, Junko, c/o Central Research Laboratories
Mitsubishi Rayon Co.,Ltd., 20-1 Miyuki-cho
Otake-shi, Hiroshima-ken(JP)

(74) Representative: TER MEER - MÜLLER - STEINMEISTER & PARTNER
Mauerkircherstrasse 45
D-8000 München 80(DE)

(54) **Process for producing metal oxide-based polycondensation powders.**

(57) Disclosed is a process for producing a metal oxide-based polycondensation powder which comprises cohydrolyzing (a) a silane compound having one or more alkoxyl groups and containing a polymerizable unsaturated bond and (b) a metal alkoxide, in a flowing aqueous medium. Moreover, if this process is carried out in a flowing aqueous medium having an inorganic powder dispersed therein, there is obtained a composite powder having polymerizable unsaturated groups and containing the inorganic powder.

EP 0 399 148 A2

# Process for Producing Powders

This invention relates to a process for the production of metal oxide-based polycondensation powders having polymerizable unsaturated groups which is based on the cohydrolysis of a metal alkoxide and a silane compound, as well as a process for the production of composite powders comprising a metal oxide and an inorganic compound and having polymerizable unsaturated groups.

Glass and ceramics are usually produced by heating raw materials to form a melt or a sintered body, and then cooling it. However, a production process not involving a melting step has also be proposed. This process comprises hydrolyzing a metal alkoxide of the general formula $M^{n+}(OR)_n$ where M is a metallic element and R is an alkyl group. Since the alkoxyl group, $OR^-$, present in the molecule thereof is a strong Lewis base, it can readily be hydrolyzed ($OR^- + H_2O \rightarrow OH^- + ROH$). The resulting hydrolyzate condenses to form an aqueous sol-like material (i.e., a suspension of very fine colloidal particles in water) or a jellylike or gelatinous gel-like material (i.e., a wet gel). These materials can be made into glass or sintered to form ceramics. This process has the following advantages over the conventional melting process.

(1) Since glass and ceramics can be produced at low temperatures, a saving of energy can be effected. Moreover, it becomes possible to produce glass and ceramics of new composition which have a high melting point and have hence been difficult to produce by the melting process.

(2) Raw materials can readily be purified by distillation or the like. Moreover, since this hydrolysis process is based on a liquid-phase reaction, it is possible to produce glass and ceramics having excellent homogeneity. Accordingly, this process are being extensively studied as a technique for the development of new materials.

According to this technique, glass and ceramics having the form of blocks, plates, fibers, thin films and the like are now being produced. However, glass and ceramics in powder form cannot be produced simply by the above-described process. Specifically, they are being produced by mechanically grinding a glass or ceramic product obtained as above (mechanical grinding method), by spraying a wet gel and drying it while it is in the form of a spray (spray drying method), or by dispersing an aqueous sol-like material in a substantially water-immiscible liquid (such as toluene, paraffin oil or spindle oil) and converting it to a gel in that state (oil droplet process).

However, the mechanical grinding method and the oil droplet method have the disadvantage that the product is subject to contamination with impurities originating from the equipment or medium and this may impair its homogeneity characteristic of the metal alkoxide hydrolysis process.

Moreover, the above-described three methods have low production efficiency when viewed from the standpoint of grinding, cleaning or energy consumption. Thus, they involve high production costs and are hence uneconomical.

Furthermore, the spray drying method and the oil droplet method have the disadvantage of requiring large-capacity equipment and hence compeling a considerable plant investment.

Conventionally, the development of composite materials have been actively carried on. However, inorganic compounds and organic polymers have substantially different properties. Thus, they lack interfacial affinity because of their poor compatibility and their substantial differences in eleastic modulus and the like. Accordingly, mere blending of an organic polymer and an inorganic compound involves several problems preventing the production of satisfactory composite materials. Specifically, such mere blending fails to use an inorganic compound in high proportions, fails to achieve uniform dispersion, and fails to produce a composite material having high strength.

As attempts to improve the interfacial affinity between an organic polymer and an inorganic compound and thereby solve the above-described problems, a number of methods for the surface modification of inorganic powders have been proposed. They include, for example, the mechanochemical method in which an inorganic compound is ground in the presence of a reactive monomer to graft an organic polymer thereon, and the irradiation method in which an inorganic compound is irradiated with a high-energy radiation to graft an organic polymer thereon.

However, the above-described mechanochemical method is based on the principle that an inorganic compound is ground to produce radicals on its surfaces and thereby initiate radical polymerization. Thus, this method has the disadvantages that the resulting product has only a low degree of grafting, that it is difficult to control the grafting, and that a reaction time of as long as several days is required. On the other hand, the irradiation method has the disadvantage of requiring a very expensive apparatus for generating the radiation.

It is an object of the present invention to provide a process for the production of metal oxide-based polycondensation powder containing little foreign matter.

It is another object of the present invention to provide a process for the production of metal oxide-based polycondensation powder which process makes it possible to produce such powder easily without using any expensive grinding machine or water-immiscible liquid.

It is still another object of the present invention to provide a process for the production of metal oxide-based polycondensation powder having polymerizable unsaturated groups.

It is a further object of the present invention to provide a process for the production of composite powder comprising a metal oxide and an inorganic compound and having a polymerizable unsaturated group which process makes it possible to produce such powder while controlling the reaction easily and without using any expensive equipment.

According to one aspect of the present invention, there is provided a process for the production of metal oxide-based polycondensation powder which comprises cohydrolyzing (a) a silane compound having one or more alkoxyl groups and containing a polymerizable unsaturated bond and (b) a metal alkoxide, in a flowing aqueous medium.

According to another aspect of the present invention, there is provided a process for the production of composite powder having a polymerizable unsaturated group which comprises cohydrolyzing (a) a silane compound having one or more alkoxyl groups and containing a polymerizable unsaturated bond and (b) a metal alkoxide, in a flowing aqueous medium having an inorganic powder dispersed therein.

As used herein, the term "flowing aqueous medium" means an aqueous medium flowing in a state of turbulent flow (i.e., in such a state of flow that various parts of the fluid are intermingling irregularly) or in a state of steady flow in which the velocity and pressure at each point of the flow are always varying irregularly about the average values thereof. Such a state of flow may be created by any of various common agitation techniques including, for example, agitation with an agitating blade or a rotor, and agitation with a jet of a fluid.

The metal alkoxide used in the present invention can be any metal alkoxide that is usually subjected to hyhdrolysis in the manufacture of glasses and ceramics. Specific examples of the metal constituting the metal alkoxide include Li, Mg, Ca, Sr, Ba, Al, Ga, In, Si, Ge, Sn, Pb, As, Sb, S, Te, Y, La, Ti, Zr, Hf, V, Nb, Ta, W, Mn, Fe, Co, Ni, Pd, Cu and Zn. The alkoxyl group constituting the metal alkoxide may be one derived from an alcohol having ten or less carbon atoms and preferably one derived from an alcohol having five or less carbon atoms. Useful metal alkoxides are those in which all valence or valences of the metal are satisfied by an alkoxyl group or groups. Preferred examples of such metal alkoxides include lithium methoxide, magnesium dimethoxide, aluminum tri-tert-butoxide, aluminum triisobutoxide, aluminum triisopropoxide, calcium di-n-propoxide, tetraethyl titanate, tetraisopropyl titanate, tetraisobutyl titanate, iron trismethoxide, iron trisethoxide, copper diisopropoxide, gallium trisethoxide, yttrium trisisopropoxide, tetraethyl zirconate, tetra-n-propyl zirconate, tetra-n-butyl zirconate, indium trisisopropoxide, tin tetraisopropoxide, antimony di-n-butoxide, barium dimethoide, lanthanum trisisopropoxide, hafnium tetraisopropoxide, hafnium tetra-tert-pentoxide, tetraethoxygermanium, tetramethoxysilane, tetraethoxysilane, tetra-n-propoxysilane, and tetra-n-butoxysilane.

The polymerizable unsaturated bond-containing silane compound used in the present invention can be any silane compound that has one or more alkoxyl groups in its molecule and containing a polymerizable unsaturated bond. However, silane compounds having two or three alkoxyl groups in the molecule are preferred because of their good cohydrolyzability with metal alkoxides. The term "polymerizable unsaturated bond" as used herein denotes, for example, ethylenically unsaturated bonds including the vinyl group. Specific examples of such silane compounds include γ-methacryloyloxypropyltrimethoxysilane [$CH_2=C(CH_3)CO_2(CH_2)_3Si(OCH_3)_3$], γ-methacryloyloxypropyldimethoxymethylsilane [$CH_2=C(CH_3)CO_2(CH_2)_3SiCH_3(OCH_3)_2$], vinyltris (β-methoxyethoxy)silane [$CH_2=CHSi(OCH_2CH_2OCH_3)_3$], vinyltriethoxysilane [$CH_2=CHSi(OC_2H_5)_3$], γ-glycidoxy -3,3-dimethyl-1-propenyltrimethoxysilane

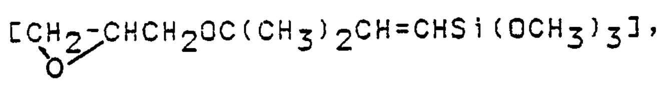

vinyltrimethoxysilane [$CH_2=CHSi(OCH_3)_3$] and γ-methacryloyloxypropyltris(β-methoxyethoxy)silane [$CH_2=C(CH_3)CO_2(CH_2)_3Si(OC_2H_4OCH_3)_3$]. Among these silane compounds, γ-methacryloyloxypropyltrimethoxysilane is preferred because it has excellent cohydrolyzability with metal alkoxides in an aqueous system, produces a significant particle-forming effect, and provides an excellent interfacial interaction between the inorganic powder and the product.

The inorganic powder used in the present invention can be any inorganic material that is substantially

stable in water. Useful inorganic powders include, for example, metals and nonmetals: oxides, hydroxides, carbides, nitrides, chlorides, sulfates, sulfites, carbonates, phosphates, silicates and double salts thereof; mixtures of the foregoing; and glasses. Especially preferred are inorganic powders which are insoluble or hardly soluble in water. Specific examples thereof include aluminum, aluminum oxide, aluminum hydroxide, silicon dioxide, silicon carbide, silicon nitride, white carbon, zirconium oxide, zirconium nitride, magnesium oxide, calcium sulfite, calcium sulfate, barium sulfate, barium oxide, antimony trioxide, calcium carbonate, titanium oxide, quartz, silica, talc, clay, carbon black, calcium phosphate, apatite, hydroxyapatite, glasses containing barium or strontium, iron and its alloys, iron oxide, nickel, zinc, copper, brass, silver, tin oxide, indium oxide, carbon, graphite, mica, gypsum, chromium, and milled carbon or glass fibers.

The inorganic powder used in the present invention may have any desired particle diameter, provided that it can be dispersed in water by agitation or other suitable means. From the viewpoint of the combination of the metal alkoxide and the inorganic powder, it is preferable to select a metal alkoxide and an inorganic powder in such a way that the metallic element constituting the metal alkoxide is the same as the metallic element forming the principal constituent of the inorganic powder. By way of example, inorganic materials such as quartz and silicon dioxide may preferably be useld in combination with metal alkoxides such as tetramethoxysilane and tetraethoxysilane. This is becasue such a combination enhances the adhesion between the inorganic powder and the metallic oxide layer formed on its surface, improves the recoverability of the resulting composite powder, and imparts a well-matched refractive index to the interface between the inorganic powder and the metallic oxcide.

In the practice of the present invention, the above-described silane compound and metal alkoxide are subjected to cohydyrolysis. This cohydrolysis can be accelerated by means of an acid or alkaline catalyst. As the acid catalyst, there may used any of various substances acting as acids. Useful acids include, for example, inorganic acids such as hydrochloric acid, sulfuric acid and nitric acid, and organic acids such as formic acid and acetic acid. As the alkaline catalyst, there may be used any of various alkalis including, for example, ammonium hydroxide, aqueous ammonia, sodium hydroxide and potassium hydroxide.

From the viewpoints of powder forming properties, filterability of the resulting powder during its recovery, and the like, it is preferable to use the polymerizable unsaturated bond-containing silane compound and the metal alkoxide in a weight ratio ranging from 5:95 to 85:15, and more preferably from 10:90 to 60:40.

When the above-defined silane compound and metal alkoxide are cohydrolyzed in a flowing aqueous medium as taught by the present invention, there is obtained a polycondensation powder comprising the resulting metal oxide and the silane compound containing polymerizable unsaturated gtroups. Although the average particle diameter of this powder depends on reaction conditions such as the flowing state of the aqueous medium, the concentration of the catalyst and the polycondensation temperature, it is greatly influenced by the ratio of the silane compound to the metal alkoxide. Specifically, the average particle diameter of the resulting powder becomes larger as the proportion of the silane compound is increased.

The amount of inorganic powder used can vary widely. Specifically, the weight ratio of the total amount of the silane compound and the metal alkoxide to the inorganic powder can range from about 500:1 to about 1:500 and preferably from about 50:1 to about 1:50.

The aqueous medium used in the present invention can preferably be deionized water. The amount of the medium used may be suitably chosen in such a range that it is not less than the stoichiometric amount required for the hydrolysis of the metal alkoxide and the silane compound and that the above-described flowing state of the reaction system can be maintained. However, the weight ratio of the total amount of the metal alkoxide and the silane compound to the water is preferably in the range of 5:95 to 70:30.

Where the process of the present invention is carried out in the presence of an inorganic powder, the weight ratio of the total amount of the metal alkoxide, the silane compound and the inorganic powder to the water is preferably in the range of 5:95 to 70:30.

Where an acid catalyst is used to accelerate the cohydrolysis, it is preferably used in such an amount that the pH of the reaction system is in the range of about 2 to 9.

Although the reaction temperature may vary according to activity of the metal alkoxide used, it is generally in the range of 10 to 100 °C and preferably in the range of 20 to 90 °C. The reaction time may also vary according to the activity of the metal alkoxide used, the reaction temperature, and the like. However, it generally ranges from 30 minutes to about 48 hours.

Generally, when a metal alkoxide is hydrolyzed, there is obtained a so-called sol-like material comprising a suspension of very fine colloidal particles of metal oxide in the liquid. As the reaction proceeds, the reaction mixture forms a block of jellylike or gelatinous gel, whether inorganic powder is dispersed in the liquid or not. In any event, it is impossible to obtain a powdery product. However, when the above-described specific metal alkoxide and silane compound are cohydrolyzed in the absence of any

inorganic powder, there is obtained a so-called wet gel powder. By drying the resulting powder at a temperature of about 10 to 300 °C and preferably about 50 to 200 °C, it can be converted to a dry gel powder having polymerizable unsaturated groups, without undergoing agglomeration.

On the other hand, when the metal alkoxide and the silane compound are cohydrolyzed in the presence of an inorganic powder, their cohydrolysis product deposits on the inorganic powder and unites firmly therewith to form a composite powder. In this composite powder, the polycondensation product obtained by the sol-gel process is united with the inorganic powder, so that the particle diameter of the composite powder is somewhat larger than that of the inorganic powder used. Thus, unlike the system containing no inorganic powder, the particle diameter of the resulting composite powder depends on that of the inorganic powder used and does not differ substantially therefrom. The composite powder produced by the process of the present invention is characterized in that its wettability by a resin is markedly improved.

By drying the composite powder resulting from the above-described cohydrolysis, at a temperature of about 10 to 300 °C and preferably about 50 to 200 °C, it can be converted to a dry composite powder having polymerizable unsaturated groups, without undergoing agglomeration.

By utilizing the polymerizable unsaturated groups contained in the aforesaid dry gel powder and dry composite powder, they can be copolymerized with organic monomers to form organic-inorganic composite materials. Alternatively, they can be fired at an appropriate temperature to form glass or ceramic powders.

The firing is preferably carried out at a temperature of 400 to 1200 °C for a period of 4 hours or more. This firing will not cause the particles of the powder to be sintered.

The present invention is further illustrated by the following examples.

In these excamples, the powder formed as a result of the reaction was separated and recovered from the aqueous medium by suction filtration through filter paper (QUALITATIVE:2, manufactured by Toyo Filter Paper Co., Ltd.) with the aid of an aspirator. Then, its recoverability was evaluated on the basis of the following rating system:

◎ = The separation of the product from the aqueous medium was completed within an hour.
O = More than one hour was required for the separation.
X = The product was in the form of a pudding or block, and no powder was obtained.

Compressive strength was measured by preparing a cylindrical hardened specimen having a diameter of 4 mm and a height of 8 mm and subjecting it to a compression test at a compression rate of 2.5 mm/min with an Instron type universal tester (Model IS-500, manufactured by Shimazu Seisakusho).

Diametral tensile strength was measured by preparing a cylindrical hardened specimen having a diameter of 6 mm and a height of 6 mm, disposing it sideways between the pressing plates of the aforesaid Instron type universal tester, and subjecting it to a compression test at a compression rate of 2.5 mm/min.

Example 1

A 500-ml four-necked flask fitted with a cooling coil, a stirring rod and a thermocouple for sensing the internal temperature was successively charged with 20 g of tetramethoxysilane [$Si(OCH_3)_4$] as a metal alkoxide, 12 g of γ-methacryloyloxypropyltrimethoxysilane [$CH_2=C(CH_3)CO_2(CH_2)_3Si(OCH_3)_3$] as a silane compound, and 252 g of deionized water. The flask was heated while stirring until its internal temperature reached 80 °C. After it was confirmed that the temperature of 80 °C was reached, 0.024 g of a 36% aqueous solution of hydrochlor ic acid was added and the reaction was continued for 8 hours under the same conditions. The pH of this reaction system ranged from 5.1 to 6.1.

When the stirring was stopped after completion of the reaction, the reaction product precipitated. This precipitate was collected by filtration, washed with acetone, and then dried in a superheated steam dryer at 105 °C for 12 hours. As a result, there was obtained 25 g of a powder having an average particle diameter of 31.7 µm. When this powder was analyzed by infrared absorption spectroscopy, an absoirption band indicating the presence of a carbon-to-carbon double bond was observed in the vicinity of 1640 $cm^{-1}$.

Comparative Example 1

The procedure of Example 1 was repeated except that no silane compound was used. Although various stirring conditions were employed, only a gelatinous gel-like material was formed in all cases, and no powder was obtained.

Examples 2 and 3

The procedure of Example 1 was repeated except that the amounts of metal alkoxide and silane compound used were changed as shown in Table 1. The results thus obtained, together with the results of Example 1 and Comaprative Example 1, are shown in Table 1. It can be seen from these results that the average particle diameter of the resulting powder varies according to the proportion of the silane compound.

Table 1

| | Tetramethoxysilane | γ-Methacryloyloxypropyltrimethoxysilane | Recoverability | Yield (g) | Average particle diameter (μm) | Form |
|---|---|---|---|---|---|---|
| Example 1 | 20 g | 12 g | ◎ | 25 | 31.7 | Powder |
| Example 2 | 24 g | 8 g | ◎ | 25 | 13.6 | Powder |
| Example 3 | 26 g | 6 g | ◎ | 24 | 5.3 | Powder |
| Comparative Example 1 | 20 g | - | × | - | -- | Block |

Examples 4-23

The procedure of Example 1 was repeated except that each of the compounds shown in Table 2 was used as the metal alkoxide. The results thus obtained are shown in Table 2.

Table 2

| Example | Metal alkoxide | Recoverability | Yield (g) | Average particle diameter (μm) | Form |
|---|---|---|---|---|---|
| 4 | $Si(OC_2H_5)_4$ | ◎ | 29 | 10.0 | Powder |
| 5 | $Si(O\text{-}n\text{-}C_3H_7)_4$ | ◎ | 27 | 11.2 | Powder |
| 6 | $Si(O\text{-}n\text{-}C_4H_9)_4$ | ◎ | 27 | 9.6 | Powder |
| 7 | $Al(O\text{-}i\text{-}C_3H_7)_3$ | ◎ | 28 | 20.5 | Powder |
| 8 | $Al(O\text{-}i\text{-}C_4H_9)_3$ | ◎ | 28 | 19.0 | Powder |
| 9 | $Al(O\text{-}t\text{-}C_4H_9)_3$ | ◎ | 25 | 20.0 | Powder |
| 10 | $Ti(OC_2H_5)_4$ | ◎ | 30 | 17.8 | Powder |
| 11 | $Ti(O\text{-}i\text{-}C_3H_7)_4$ | ◎ | 26 | 15.4 | Powder |
| 12 | $Ti(O\text{-}n\text{-}C_4H_9)_4$ | ◎ | 26 | 16.5 | Powder |
| 13 | $Zr(OC_2H_5)_4$ | ◎ | 30 | 13.0 | Powder |
| 14 | $Zr(O\text{-}N\text{-}C_3H_7)_4$ | ◎ | 27 | 13.2 | Powder |
| 15 | $Zr(O\text{-}n\text{-}C_4H_9)_4$ | ◎ | 27 | 12.5 | Powder |
| 16 | $Sb(O\text{-}n\text{-}C_4H_9)_2$ | ○ | 21 | 14.0 | Powder |
| 17 | $Fe(OC_2H_5)_3$ | ◎ | 20 | 48.8 | Powder |
| 18 | $Cu(O\text{-}i\text{-}C_3H_7)_2$ | ◎ | 19 | 42.0 | Powder |
| 19 | $Ga(OC_2H_5)_3$ | ◎ | 25 | 40.9 | Powder |
| 20 | $Y(O\text{-}i\text{-}C_3H_7)_3$ | ◎ | 23 | 45.5 | Powder |
| 21 | $Sn(O\text{-}i\text{-}C_3H_7)_4$ | ○ | 23 | 63.2 | Powder |
| 22 | $Ba(OCH_3)_2$ | ◎ | 31 | 56.3 | Powder |
| 23 | $Hf(O\text{-}i\text{-}C_3H_7)_4$ | ○ | 19 | 50.2 | Powder |

Examples 24 and 25 and Comparative Example 2

The procedure of Example 1 was repeated except that each of the compounds shown in Table 3 was used as the silane compound. The results thus obtained are shown in Table 3.

Table 3

| | Silane compound | Recoverability | Yield (g) | Average particle diameter (μm) | Form |
|---|---|---|---|---|---|
| Example 24 | Allyltriethoxysilane | ○ | 19 | 2.3 | Powder |
| Example 25 | Vinyltriethoxysilane | ○ | 21 | 3.0 | Powder |
| Comparative Example 2 | Pentyltriethoxysilane | × | - | -- | Block |

Examples 26-35

The procedure of Example 1 was repeated except that each of the mixtures shown in Table 4 was used as the metal alkoxide. The results thus obtained are shown in Table 4.

Table 4

| Example | Metal alkoxide | Amount used (g) | Recoverability | Yield (g) | Average particle diameter (μm) | Form |
|---|---|---|---|---|---|---|
| 26 | $Si(OCH_3)_4$<br>$Al(O\text{-}i\text{-}C_4H_4)_3$ | 18<br>6 | ◎ | 25 | 14.9 | Powder |
| 27 | $Si(OCH_3)_4$<br>$Al(O\text{-}i\text{-}C_4H_9)_3$ | 12<br>12 | ◎ | 27 | 16.3 | Powder |
| 28 | $Si(OCH_3)_4$<br>$Al(O\text{-}i\text{-}C_4H_9)_3$ | 6<br>18 | ◎ | 29 | 17.6 | Powder |
| 29 | $Si(OCH_3)_4$<br>$Ti(OC_2H_5)_4$ | 12<br>12 | ◎ | 27 | 15.7 | Powder |
| 30 | $Si(OCH_3)_4$<br>$Zr(OC_2H_5)_4$ | 12<br>12 | ◎ | 29 | 13.3 | Powder |
| 31 | $Si(OCH_3)_4$<br>$Sb(O\text{-}n\text{-}C_4H_9)_2$ | 12<br>12 | ○ | 26 | 13.8 | Powder |

- to be cont'd. -

Table 4 (cont'd.)

| | | | | | | |
|---|---|---|---|---|---|---|
| 32 | $Si(OCH_3)_4$<br>$Hf(O\text{-}i\text{-}C_3H_7)_4$ | 12<br>12 | ○ | 25 | 31.9 | Powder |
| 33 | $Al(O\text{-}i\text{-}C_4H_9)_3$<br>$Ti(OC_2H_5)_4$ | 12<br>12 | ◎ | 30 | 18.4 | Powder |
| 34 | $Ti(OC_2H_5)_4$<br>$Zr(OC_2H_5)_4$ | 12<br>12 | ◎ | 28 | 15.4 | Powder |
| 35 | $Al(O\text{-}i\text{-}C_4H_9)_3$<br>$Zr(OC_2H_5)_4$ | 12<br>12 | ◎ | 30 | 16.0 | Powder |

Examples 36-41

The procedures of Exlamples 11, 15 and 16 were repeated except that the ratio of the metal alkoxide to the silane compound was changed as shown in Table 5. The recoverabilities of these examples were very

good. The results thus obtained, together with those of Examples 11, 15 and 16, are shown in Table 5.

Table 5

| Example | Metal alkoxide | Amount used | γ-Methacryloyloxypropyltrimethoxysilane | Average particle diameter (μm) |
|---|---|---|---|---|
| 36 | $Ti(O\text{-}i\text{-}C_3H_7)_4$ | 20 g | 12 g | 35.9 |
| 11 | $Ti(O\text{-}i\text{-}C_3H_7)_4$ | 24 g | 8 g | 15.4 |
| 37 | $Ti(O\text{-}i\text{-}C_3H_7)_4$ | 26 g | 6 g | 6.0 |
| 38 | $Zr(O\text{-}n\text{-}C_4H_9)_4$ | 20 g | 12 g | 29.1 |
| 15 | $Zr(O\text{-}n\text{-}C_4H_9)_4$ | 24 g | 8 g | 12.5 |
| 39 | $Zr(O\text{-}n\text{-}C_4H_9)_4$ | 26 g | 6 g | 4.9 |
| 40 | $Sb(O\text{-}n\text{-}C_4H_9)_2$ | 20 g | 12 g | 32.6 |
| 16 | $Sb(O\text{-}n\text{-}C_4H_9)_2$ | 24 g | 8 g | 14.0 |
| 41 | $Sb(O\text{-}n\text{-}C_4H_9)_2$ | 26 g | 6 g | 5.5 |

Example 42

When each of the powders obtained in Examples 1-3 was heat-treated at 700 °C for 12 hours, neither destruction nor sintering of the particles occurred. Thus, there were obtained metal oxide-based polycondensation powders containing no organic component.

Example 43

A 500-ml four-necked flask fitted with a cooling coil, a stirring rod and a thermocouple for sensing the internal temperature was successively charged with 28 g of tetramethoxysilane [$Si(OCH_3)_4$] as a metal alkoxide, 4 g of γ-methacryloyloxypropyltrimethoxysilane [$CH_2=C(CH_3)CO_2(CH_2)_3So(OCH_3)_3$] as a silane compound, 32 g of quartz powder (with an average particle diameter of about 3.5 μm), and 252 g of a deionized water. The flask was heated while stirring until its internal temperature reached 80 °C. After it was confirmed that the temperature of 80 °C was reached (about 45 minutes after charging), 0.024 g of a 36% aqueous solution of hydrochloric acid was added and the reaction was continued for 8 hours under the same conditions. The pH of this reaction system ranged from 5.4 to 6.3.

When the stirring was stopped after completion of the reaction, the reaction product precipitated. This precipitate was collected by filtration, washed with acetone, and then dried in a superheated steam dryer at 105 °C for 12 hours. As a result, there was obtained 58 g of a composite powder. When this powder was analyzed by infrared absorption spectroscopy, an absorption band indicating the presence of a carbon-to-carbon double bond was observed in the vicinity of 1640 $cm^{-1}$.

Comparative Example 3

The procedure of Example 43 was repeated except that no silane compound was used. Although various stirring conditions were employed, only a gelatinous gel-like material having the inorganic powder dispersed therein was formed in all cases, and no powder was obtained.

Examples 44-46

The procedure of Example 43 was repeated except that the amounts of metal alkoxide and silane compound used were changed as shown in Table 6. The results thus obtained, together with the results of Example 43 and Comparative Example 3, are shown in Table 6.

Table 6

| | Tetramethoxysilane | γ-Methacryloyloxypropyltrimethoxysilane | Recoverability | Yield (g) | Form |
|---|---|---|---|---|---|
| Example 43 | 28 g | 4 g | ◎ | 58 | Powder |
| Example 44 | 24 g | 8 g | ◎ | 58 | Powder |
| Example 45 | 20 g | 12 g | ◎ | 57 | Powder |
| Example 46 | 6 g | 2 g | ◎ | 39 | Powder |
| Comparative Example 3 | 32 g | - | × | - | Block |

Examples 47-66

The procedure of Example 44 was repeated except that each of the compounds shown in Table 7 was used as the metal alkoxide. The results thus obtained are shown in Table 7.

Table 7

| Example | Metal alkoxide | Recoverability | Yield (g) | Form |
|---|---|---|---|---|
| 47 | $Si(OC_2H_5)_4$ | ◎ | 59 | Powder |
| 48 | $Si(O\text{-}n\text{-}C_3H_7)_4$ | ◎ | 57 | Powder |
| 49 | $Si(O\text{-}n\text{-}C_4H_9)_4$ | ◎ | 57 | Powder |
| 50 | $Al(O\text{-}i\text{-}C_3H_7)_3$ | ◎ | 58 | Powder |
| 51 | $Al(O\text{-}i\text{-}C_4H_9)_3$ | ◎ | 58 | Powder |
| 52 | $Al(O\text{-}t\text{-}C_4H_9)_3$ | ◎ | 55 | Powder |
| 53 | $Ti(OC_2H_5)_4$ | ◎ | 60 | Powder |
| 54 | $Ti(O\text{-}i\text{-}C_3H_7)_4$ | ◎ | 56 | Powder |
| 55 | $Ti(O\text{-}i\text{-}C_4H_9)_4$ | ◎ | 56 | Powder |
| 56 | $Zr(OC_2H_5)_4$ | ◎ | 60 | Powder |
| 57 | $Zr(O\text{-}n\text{-}C_3H_7)_4$ | ◎ | 57 | Powder |
| 58 | $Zr(O\text{-}n\text{-}C_4H_9)_4$ | ◎ | 57 | Powder |
| 59 | $Sb(O\text{-}n\text{-}C_4H_9)_2$ | ○ | 51 | Powder |
| 60 | $Fe(OC_2H_5)_3$ | ◎ | 50 | Powder |
| 61 | $Cu(O\text{-}i\text{-}C_3H_7)_2$ | ◎ | 49 | Powder |
| 62 | $Ga(OC_2H_5)_3$ | ◎ | 55 | Powder |
| 63 | $Y(O\text{-}i\text{-}C_3H_7)_3$ | ◎ | 53 | Powder |
| 64 | $Sn(O\text{-}i\text{-}C_3H_7)_4$ | ○ | 53 | Powder |
| 65 | $Ba(OCH_3)_2$ | ◎ | 61 | Powder |
| 66 | $Hf(O\text{-}i\text{-}C_3H_7)_4$ | ○ | 49 | Powder |

Examples 67 and 68 and Comparative Example 4

The procedure of Example 44 was repeated except that each of the compounds shown in Table 8 was used as the silane compound. The results thus obtained are shown in Table 8.

Table 8

| | Silane compound | Recoverability | Yield (g) | Form |
|---|---|---|---|---|
| Example 67 | Allyltriethoxysilane | ○ | 49 | Powder |
| Example 68 | Vinyltriethoxysilane | ○ | 51 | Powder |
| Comparative Example 4 | Pentyltriethoxysilane | × | - | Block |

Examples 69-78

The procedure of Example 44 was repeated except that each of the mixtures shown in Table 9 was used as the metal alkoxide. The results thus obtained are shown in Table 9.

Table 9

| Example | Metal alkoxide | Amount used (g) | Recoverability | Yield (g) | Form |
|---|---|---|---|---|---|
| 69 | $Si(OCH_3)_4$ | 18 | ◎ | 55 | Powder |
| | $Al(O\text{-}i\text{-}C_4H_9)_3$ | 6 | | | |
| 70 | $Si(OCH_3)_4$ | 12 | ◎ | 57 | Powder |
| | $Al(O\text{-}i\text{-}C_4H_9)_3$ | 12 | | | |
| 71 | $Si(OCH_3)_4$ | 6 | ◎ | 59 | Powder |
| | $Al(O\text{-}i\text{-}C_4H_9)_3$ | 18 | | | |
| 72 | $Si(OCH_3)_4$ | 12 | ◎ | 57 | Powder |
| | $Ti(OC_2H_5)_4$ | 12 | | | |
| 73 | $Si(OCH_3)_4$ | 12 | ◎ | 59 | Powder |
| | $Zr(OC_2H_5)_4$ | 12 | | | |
| 74 | $Si(OCH_3)_4$ | 12 | ○ | 56 | Powder |
| | $Sb(O\text{-}n\text{-}C_4H_9)_2$ | 12 | | | |
| 75 | $Si(OCH_3)_4$ | 12 | ○ | 55 | Powder |
| | $Hf(O\text{-}i\text{-}C_3H_7)_4$ | 12 | | | |
| 76 | $Al(O\text{-}i\text{-}C_4H_9)_3$ | 12 | ◎ | 60 | Powder |
| | $Ti(OC_2H_5)_4$ | 12 | | | |
| 77 | $Ti(OC_2G_5)_4$ | 12 | ◎ | 58 | Powder |
| | $Zr(OC_2H_5)_4$ | 12 | | | |
| 78 | $Al(O\text{-}i\text{-}C_4H_9)_3$ | 12 | ◎ | 60 | Powder |
| | $Zr(OC_2H_5)_4$ | 12 | | | |

Examples 79-103

The procedure of Example 44 was repeated except that each of the materials shown in Table 10 was used as the inorganic powder. The results thus obtained are shown in Table 10.

Table 10

| Example | Inorganic powder | Average particle diameter (μm) | Recoverability | Yield (g) | Form |
|---|---|---|---|---|---|
| 79 | Amorphous silica | 0.04 | ◎ | 62 | Powder |
| 80 | Silicon carbide | 3 | ◎ | 60 | Powder |
| 81 | Silicon nitride | 4 | ◎ | 60 | Powder |
| 82 | Aluminum oxide | 7-8 | ◎ | 53 | Powder |
| 83 | Zirconium oxide | 5 | ◎ | 56 | Powder |
| 84 | Titanium oxide | 0.5-1 | ◎ | 50 | Powder |
| 85 | Calcium carbonate | 2 | ◎ | 49 | Powder |
| 86 | Antimony trioxide | ~1 | ◎ | 50 | Powder |
| 87 | Barium sulfate | 3 | ◎ | 53 | Powder |
| 88 | Barium glass | 8-9 | ◎ | 60 | Powder |
| 89 | Carbon black | 0.008 | ◎ | 51 | Powder |
| 90 | Talc | ~1 | ◎ | 58 | Powder |
| 91 | Hydroxyapatite | 14 | ◎ | 57 | Powder |
| 92 | Mica | 10-12 | ◎ | 57 | Powder |
| 93 | Silver | 3-4 | ◎ | 50 | Powder |
| 94 | Nickel | 3-4 | ◎ | 52 | Powder |
| 95 | Zinc | 3-4 | ◎ | 49 | Powder |
| 96 | Aluminum hydroxide | 7-8 | ◎ | 53 | Powder |
| 97 | Calcium sulfite | 7-8 | ◎ | 52 | Powder |
| 98 | Calcium chloride | 7-8 | ◎ | 48 | Powder |
| 99 | Strontium glass | 5-6 | ◎ | 59 | Powder |
| 100 | Lead glass | 5-6 | ◎ | 58 | Powder |
| 101 | Iron-nickel-chlromium alloy | 8 | ◎ | 50 | Powder |
| 102 | Graphite | 10-12 | ◎ | 52 | Powder |
| 103 | White carbon | 0.06 | ◎ | 60 | Powder |

Example 104

When each of the composite powders obtained in Examples 43-45 was heat-treated at 700° C for 12 hours, neither destruction nor sintering of the particles occurred. Thus, there were obtained fired composite powders containing no organic component.

Examples 105-107 and Comparative Examples 5 and 6

The composite powder obtained in Example 79, a mixture of polymethyl methacrylate powder (Acricon® AC, manufactured by Mitsubishi Rayon Co., Ltd.) and methyl methacrylate monomer (in a weight ratio of 2:1), triethylene glycol dimethacrylate and benzoyl peroxide were mixed according to the formulations shown in Table 11. These mixtures were charged into gypsum molds and polymerized by dry-heating at 60° C for 3 hours and then at 100° C for 1 hour. After the hardened products were immersed in water at 37° C for 24 hours, the compressive strength and diametral tensile strength thereof were measured. The results thus obtained are shown in Table 11. In Table 11 and Table 12 that will be given later, TEGDMA stands for triethylene glycol dimethacrylate.

For purposes of comparison, the procedure of Example 105 was repeated except that the composite powder was replaced by amorphous silica powder treated with a silane coupling agent (3-methacryloyloxypropyltrimethoy silane) (Comparative Example 5) or untreated amorphous silace powder (Comparative Example 6). Then, the mechanical strength values of the hardened products were measured. The results thus obtained are also shown in Table 11.

It can be seen from Table 11 that the composite materials prepared with the composite powder of the present invention had more excellent mechanical properties than those prepared with the conventional

silane-treated or untreated powder. Moreover, when the composite powder of the present invention is compounded with a liquid resin, it shows very good wettability by the resin. Thus, the composite powder of the present invention could readily be used to form composite materials. Furthermore, the hardened specimens thus obtained had a much more attractive appearance, as compared with those obtained in the comparative examples. These results indicate that the composite powder of the present invention is suitable for use as a filler for dental materials.

Examples 108-110 and Comaprative Example 7

The procedure of Example 105 was repeated except that the composite powder obtained in Example 82 was used in place of that obtained in Example 79, bisphenol A diglycidyl methacrylate was used in place of the poly methyl methacrylate/methyl methacrylate monomer mixture, and each of the formulations shown in Table 12 was employed. Then, the mechanical strength values of the resulting hardened products were measured. The results thus obtained are shown in Table 12. In Table 12, BPADGMA stands for bisphenol A diglycidyl methacrylate.

It can be seen from Table 12 that the hardened specimens of the composite material prepared with the untreated powder in Comparative Example 7 had very low strength values, whereas the hardened specimens of the composite materials prepared with the composite powder of the present invention exhibited excellent mechanical properties. Moreover, when the composite powder of the present invention is compounded with a liquid resin, it showes very good wettability by the resin. Thus, the composite powder of the present invention could readily be used to form composite materials. Furthermore, the hardened specimens thus obtained had a much more attractive appearance, as compared with those obtained in the comparative example. These results indicate that the composite powder of the present invention is suitable for use as a filler for dental materials.

Table 11

| | Inorganic powder | | Amount of PMMA/MMA mixture (parts) | Amount of TEGDMA (parts) | Amount of BPO (parts) | Mechanical strength ($kgf/cm^2$) | |
|---|---|---|---|---|---|---|---|
| | Type | Amount (parts) | | | | Compressive strength | Tensile strength |
| Example 105 | Composite powder | 1.00 | 5.70 | 0.7 | 0.032 | 2020 | 410 |
| Example 106 | Composite powder | 1.46 | 5.24 | 0.7 | 0.032 | 2100 | 418 |
| Example 107 | Composite powder | 1.92 | 4.78 | 0.7 | 0.032 | 2150 | 420 |
| Comparative Example 5 | Silane-treated powder | 1.00 | 5.70 | 0.7 | 0.032 | 1756 | 364 |
| Comparative Example 6 | Untreated powder | 1.00 | 5.70 | 0.7 | 0.032 | 1022 | 312 |

Table 12

| | Inorganic powder | | Amount of BPADGMA (parts) | Amount of TEGDMA (parts) | Amount of BPO (parts) | Mechanical strength (kgf/cm$^2$) | |
|---|---|---|---|---|---|---|---|
| | Type | Amount (parts) | | | | Compressive strength | Tensile strength |
| Example 108 | Composite powder | 4.60 | 3.06 | 1.54 | 0.023 | 950 | 250 |
| Example 109 | Composite powder | 6.11 | 1.55 | 1.54 | 0.023 | 980 | 270 |
| Example 110 | Composite powder | 7.62 | 0.04 | 1.54 | 0.023 | 990 | 285 |
| Comparative Example 7 | Untreated powder | 4.60 | 3.06 | 1.54 | 0.023 | 169 | 95 |

According to the process of the present invention, metal oxide-based polycondensation powders can be easily produced without using any expensive grinding machine or water-immisible liquid. The powders so produced contain less foreign matter, as compared with those produced by prior art processes. Moreover, novel composite ceramic powders having vinyl groups can also be produced at lower cost.

Since the powders produced by the process of the present invention have polymerizable unsaturated groups, they can be used to produce composite materials having a good affinity between the organic and inorganic comonents and having high mechanical strength, by utilizing the reactivity of the unsaturated groups. Moreover, they are also suitable for use as fillers for dental materials.

## Claims

1. A process for producing a metal oxide-based polycondensation powder which comprises cohydrolyzing (a) a silane compound having one or more alkoxyl groups and containing a polymerizable unsaturated bond and (b) a metal alkoxide, in a flowing aqueous medium.

2. A process as claimed in claim 1 wherein the alkoxyl group of the metal alkoxide is derived from an alcohol having ten or less carbon atoms.

3. A process as claimed in claim 1 wherein the silane compound has two or three alkoxyl groups in the molecule thereof.

4. A process as claimed in claim 1 wherein the silane compound is $\gamma$-methacryloyloxypropyltrimethoxysilane.

5. A process as claimed in claim 1 wherein the weight ratio of silane compound to the metal alkoxide is in the range of 5:95 to 85:15.

6. A process as claimed in claim 1 wherein the weight ratio of the total amount of the silane compound and the metal alkoxide to the aqueous medium is in the range of 5:95 to 70:30.

7. A process for producing a composite powder having a polymerizable unsaturated group which comprises cohydrolyzing (a) a silane compound having one or more alkoxyl groups and containing a polymerizable unsaturated bond and (b) a metal alkoxide, in a flowing aqueous medium having an inorganic powder dispersed therein.

8. A process as claimed in claim 7 wherein the alkoxyl group of the metal alkoxide is derived from an alcohol having ten or less carbon atoms.

9. A process as claimed in claim 7 wherein the silane compound has two or three alkoxyl groups in the molecule thereof.

10. A process as claimed in claim 7 wherein the silane compound is $\gamma$-methacryloyloxypropyltrimethoxysilane.

11. A process as claimed in claim 7 wherein the metal alkoxide and the inorganic powder are selected in such a way that the metallic element constituting the metal alkoxide is the same as the metallic element forming the principal cosntituent of the inorganic powder.

12. A process as claimed in claim 7 wherein the weight ratio of silane compound to the metal alkoxide is in the range of 5:95 to 85:15.

13. A process as claimed in claim 7 wherein the weight ratio of the total amount of the silane compound and the metal alkoxide to the inorganic powder is in the range of 500:1 to 1:500.

14. A process as claimed in claim 7 wherein the weight ratio of the total amount of the silane compound, the metal alkoxide and the inorganic powder to the aqueous medium is in the range of 5:95 to 70:30.